# EUROPEAN PATENT SPECIFICATION

(11) **EP 1 608 663 B1**
(45) Date of publication and mention of the grant of the patent: **05.11.2008**
(21) Application number: 03816507.2
(22) Date of filing: 31.03.2003
(51) Int. Cl.: C07D 519/00, A61K 31/5517, A61P 35/00

(54) **PYRROLO (2,1-C)(1,4) BENZODIAZEPINES DIMERS AS ANTITUMOUR AGENTS AND PROCESS THEREOF**
PYRROLO(2,1-C)(1,4)BENZODIAZEPIN-DIMERE ALS ANTITUMORMITTEL UND VERFAHREN DAFÜR
DIMERES DE PYRROLO(2,1-C) (1,4) BENZODIAZEPINE EN TANT QU'AGENTS ANTITUMORAUX ET PROCEDE CORRESPONDANT

(43) Date of publication of application: 28.12.2005
(73) Proprietor: Council of Scientific and Industrial Research, New Delhi 110 001 (IN)
(72) Inventor: KAMAL, Ahmed, 500 007 Andhra Pradesh (IN); REDDY, Peram, Surakattula, Murali, Mohan, 500 007 Andhra Pradesh (IN); REDDY, Depatla, Rajasekhar, 500 007 Andhra Pradesh (IN)
(74) Representative: Adam, Holger
(86) International application number: PCT/IB2003/001164
(87) International publication number: WO 2004/087716

(56) References cited:
- WO-A-93/18045
- REDDY B S P ET AL: "SYNTHETIC DNA MINOR GROOVE-BINDING DRUGS" PHARMACOLOGY AND THERAPEUTICS, ELSEVIER, GB, vol. 84, October 1999 (1999-10), pages 1-111, XP000984832 ISSN: 0163-7258
- KAMAL, A. ET AL.: "Synthesis of C-8 Alkylamino Substituted Pyrrolo(2,1-c)(1,4)benzodiazepines as Potential Anti-Cancer Agents" BIOORG. MED. CHEM. LETT., vol. 12, 2002, pages 1917-1919, XP002249903

## Description

### Field of the invention

The present invention relates to novel pyrrolo[2,1-*c*][1,4]benzodiazepines useful as potential antitumour agents. This invention relates to a process for the preparation of new pyrrolo[2,1-*c*][1,4]benzodiazepines useful as antitumour agents. More particularly, it provides a process for the preparation of 1,1'-{[(bisalkane-1,N-diyl)piperazine]dioxy} bis[(11a*S*)-7-methoxy-1, 2, 3, 11a-tetrahydro-5*H*-pyrrolo[2,1-*c*][1,4]benzodiazepin-5-one, with aliphatic chain length variations for the compounds and it also describes the anticancer (antitumour) activity. The structural formula of novel pyrrolo[2,1-*c*][1,4]benzodiazepine is as follows, wherein n = 2 to 10.

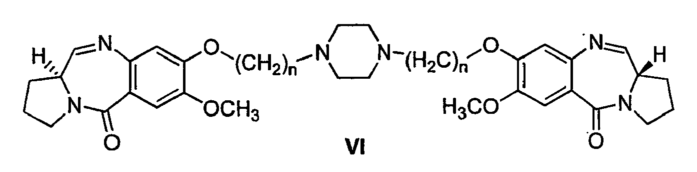

### Background and Prior art references

Pyrrolo[2,1-*c*][1,4]benzodiazepines antitumour antibiotics are commonly known as anthramycin class of compounds. In the last few years, a growing interest has been shown in the development of new pyrrolo[2,1-*c*][1,4]benzodiazepines (PBDs). These antibiotics react covalently with DNA to form an N2-guanine adduct that lies within the minor groove of duplex DNA *via* an acid-labile aminal bond to the electrophilic imine at the N10-C11 position. (Kunimoto, S. Masuda, T. Kanbayashi, N. Hamada, M. Naganawa, H. Miyamoto, M. Takeuchi, T. and Unezawa, H. J. Antibiot., 1980, 33, 665.; Kohn, K.W. and Speous, C.L. J. Mol. Biol., 1970, 51, 551.; Hurley, L.H. Gairpla, C. and Zmijewski, M. Biochem. Biophys. Acta., 1977, 475, 521.; Kaplan, D.J. and Hurley, L.H. Biochmestry, 1981, 20, 7572). The molecules have a right-handed twist, which allows them to follow the curvature of the minor groove of B-form double-stranded DNA spanning three base pairs. Recently PBD dimers have been developed that comprises two C2-exo-methylene- substituted DC-81 subunits tethered through their C-8 position via an inert propanedioxy linker. (S. J. Gregson, P. W.Howard, J. A. Hartely, N. A. Brooks, L. J Adams, T. C. Jenkins, L. R. Kelland, and D. E.Thurston. J.Med.Chem., 2001, 44, 737). A recent development has been the linking of two PBD units through their C-8 positions to give bisfunctional alkylating agents capable of cross-linking DNA (Thurston, D. E. Bose, D. S. Thomson, A. S. Howard, P. W. Leoni, A. Croker, S. J. Jenkins, T. C. Neidle, S. and Hurley, L. H. J. Org. Chem., 1996, 61, 8141-8147). Recently, a noncross-linking mixed imine-amide PBD dimers have been synthesized that have significant DNA binding ability and potent anti tumour activitiy (Kamal, A.; Laxman, N.; Ramesh, G.; Ramulu, P and Srinivas, O. US Pat. No. 636233. dt 26-03-2002.; Kamal, A.; Ramesh, G.; Laxman, N.; Ramulu, P.; Srinivas, O.; Neelima, K.; Kondapi, A. K.; Srinu, V. B.; Nagarajaram, H. M. J. Med. Chem. 2002, 45, 4679 ).

Among further prior art, WO 93/18045 discloses PBD dimers which are cytotoxic and show in vitro activity against a range of carcinoma cell lines. Praven Reddy et al. (Pharmacology & Therapeutics 1999, 84, 1-111) describe minor groove-binding drugs of the PBD family. Kamal et al. have reported monomeric 8-alkylamino substituted PBD compounds (Bioorg. Med. Chem. Lett. 2002, 12, 1917-1919).

Naturally occurring pyrrolo[2,1-*c*][1,4]benzodiazepines belong to a group of antitumour antibiotics derived from *Streptomyces* species. Recently, there is much 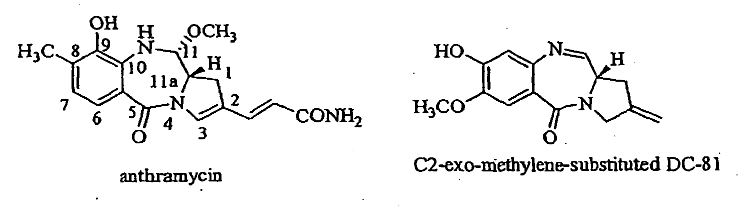 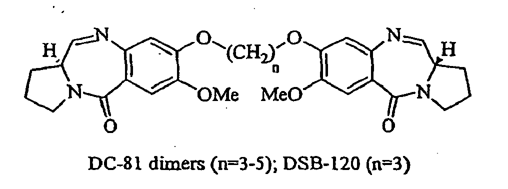 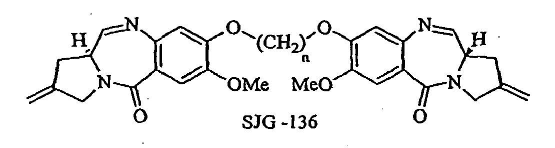 impetus for the PBD systems as they can recognize and bind to specific sequence of DNA. Examples of naturally occurring PBD's include anthramycin, DC-81, tomaymycin, sibiromycin and neothramycin.

However, the clinical efficacy for these antibiotics is hindered by several limitations, such as poor water solubility and cardiotoxicity and development of drug resistance and metabolic inactivation.

Still another embodiment, the invention provides novel pyrrolobenzodiazepine having structural formula as shown below. (n = 3)

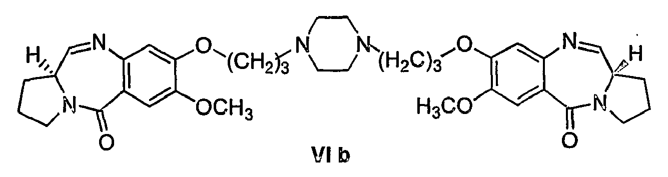

Still another embodiment, the invention provides novel pyrrolobenzodiazepine having structural formula as shown below. (n= 4)

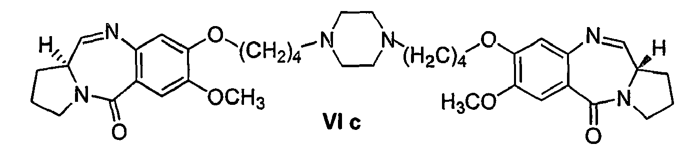

Still another embodiment, the invention provides novel pyrrolobenzodiazepine having structural formula as shown below. (n= 5)

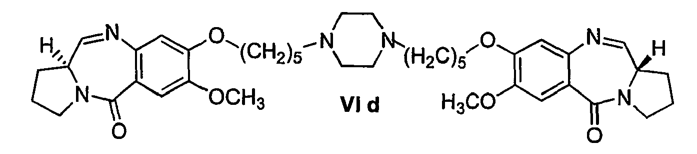

Still another embodiment, the invention provides novel pyrrolobenzodiazepine having structural formula as shown below. (n= 6)

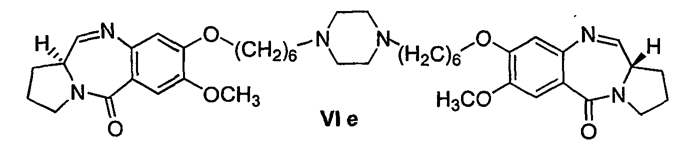

Still yet another embodiment, the invention provides novel pyrrolobenzodiazepine having structural formula as shown below. (n= 7)

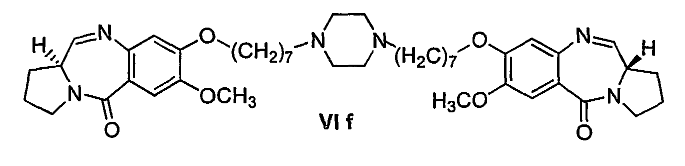

### Objects of the invention

The main object of the present invention is to provide new pyrrolo[2,1-*c*][1,4]benzodiazepines useful as antitumour agents.

Another object of the invention is to provide pharmaceutical compositions comprising novel pyrrolo[2,1-*c*][1,4]benzodiazepines useful as anti-cancer agents

Another objective of the present invention is to provide a process for the preparation of novel pyrrolo[2,1-*c*][1,4] benzodiazepines..

### Summary of the invention

Accordingly, the present invention provides novel pyrrolo[2,1-*c*][1,4]benzodiazepine of formula VI where n is 2 to 10; and a process for the preparation of the same.

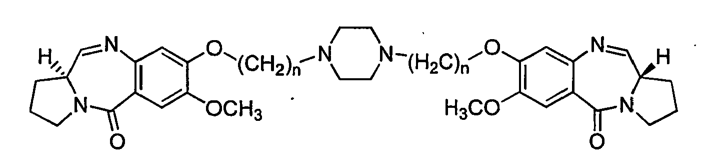

### Detailed Description of the Invention

In accordance, the present invention provides analogues of 1, 1'-{[(bisalkane-1,N-diyl)piperazine]dioxy}bis(11aS)-7-methoxy-1,2,3,11a-tetrahydro-5H-pyrrolo[2,1-*c*][1,4]benzodiazepin-5-one] of formula (VI) 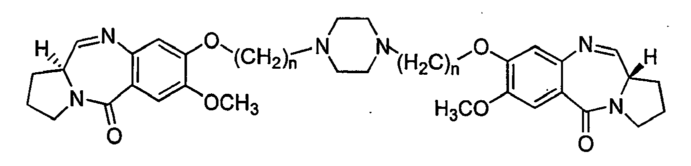 where n = 2 to 10

Another embodiment of the invention provides a novel pyrrolobenzodiazepine having structural formula as shown below. (n = 2)

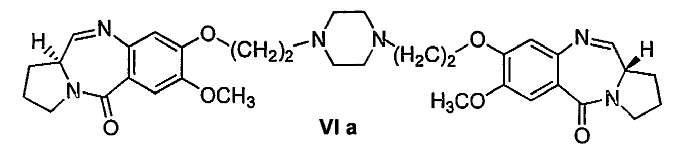

Yet another embodiment, the invention provides novel pyrrolobenzodiazepine having structural formula as shown below. (n= 8)

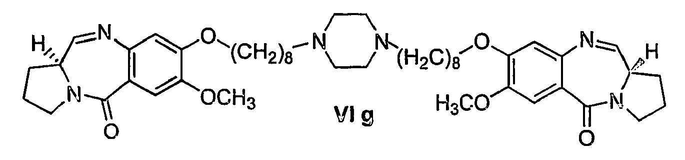

Still yet another embodiment, the invention provides novel pyrrolobenzodiazepine having structural formula as shown below. (n= 9)

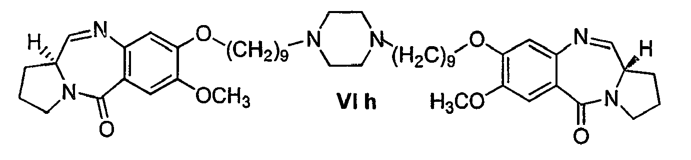

Still yet another embodiment, the invention provides novel pyrrolobenzodiazepine having structural formula as shown below. (n = 10)

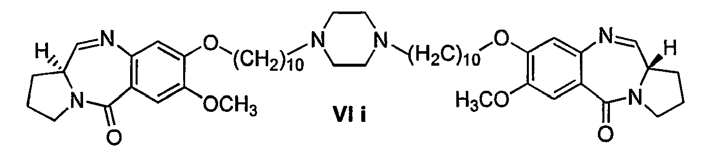

In an embodiment of the inventioin provides a process for the preparation of analogues of 1, 1'-{[(bisalkane-1,N-diyl)piperazine]dioxy}bis(11aS)-7-methoxy-1,2,3,11a-tetrahydro-5H-pyrrolo[2,1-*c*][1,4]benzodiazepin-5-one] of formula (VI), the said process comprising steps of:
a) reacting compound of formula (I) with 1,2-dibromoethane in water miscible organic solvent in presence of a base at a reflux temperature for a period of 20h to 48h,
b) pouring the reaction mixture of step (a) onto water, extracting with ethylacetate separating ethylacetate layer and discarding aqueous layer,
c) evaporating the ethylacetate layer of step (b) to obtain a residue which is further purified to obtain pure compound of formula (II),
d) providing a solution of formula (II) in a ketonic solvent in presence of a base at a reflux temperature for a period of 20 h to 48 h,
e) pouring the reaction mixture of step (d) onto water, extracting with ethylacetate, separating ethylacetate layer, evaporating ethylacetate layer to obtain a residue, purifying the residue to get compound of formula (IV),
f) dissolving compound of formula (IV) in alcohol, adding stannous chloride dihydrate, refluxing for 0.5 h to 1.5h,
g) adjusting the pH of the reaction mixture of step (f) to 8.0 using alkali bicarbonate solution,
h) extracting solution of pH 8.0 of step (g) with ethylacetate, separating ethylacetate extract, drying ethylacetate extract over anhydrous sodium sulphate, filtered and evaporated ethyl acetate solution to yield a crude compound of formula (V),
i) dissolving compound of formula (V) of step (h) in a mixture of acetonitrile/water, adding mercuric chloride, mercuric oxide and stirred for 6h to 12 h at an ambient temperature,
j) evaporating organic layer of step (i), diluting the residue with ethylacetate, adding saturated bicarbonate solution at room temperature, filtering through celite bed, washed with ethyl acetate to obtain a clear filterate; and
k) evaporating filtrate of step (j) to obtain a residue which is purified over silica gel column to yield pure compound of formula (VI).

Another embodiment of the invention, the base used is selected from a group consisting of lithium carbonate, sodium carbonate, potassium carbonate or cesium carbonate.

Another embodiment of the invention the ketonic solvent used is selected from a group consisting of acetone, methyl ethyl ketone and methyl isobutylketone.

Another embodiment of the invention the alcohol used is selected from methanol, ethanol and isopropanol, preferably methanol.

One more embodiment of the invention provides a pharmaceutical composition useful as anti-tumor agent, said composition comprising an effective amount of one or more analogues of 1, 1'-{[(bisalkane-1,N-diyl)piperazine]dioxy}bis(11aS)-7-methoxy-1,2,3,11a-tetrahydro-5H-pyrrolo[2,1-*c*][1,4]benzodiazepin-5-one] of formula (VI).

Still another embodiment the composition optionally comprising of pharmaceutically acceptable additives.

Yet another embodiment, the composition is administered to mammals including human beings.

Yet another embodiment, the composition may be administered orally, systemically or by any other conventional methods

The process for preparation of pyrrolo[2,1-*c*][1,4]benzodiazepines of formula VI of the drawing accompanying the specification wherein n is 2 to 10 which comprises: reacting (2*S*)-N-[4-hydroxy-5-methoxy-2-nitrobenzoyl]-2-carboxaldehyde diethylthioacetal of formula I with dibromoalkanes in an aprotic water miscible organic solvents like acetone, THF, and DMF in presence of a mild inorganic bases like K₂CO₃, CsCO₃ and BaCO₃ upto refluxing temperature for a period up to 48 hours, isolating (2*S*)-N-[4-(n-bromoalkoxy)-5-methoxy-2-nitrobenzoyl]pyrrolidine-2-carboxaldehyde diethyl thioacetal of formula II with piperazine of formula III in presence of mild inorganic bases like K₂CO₃, CsCO₃, and BaCO₃ and in presence of aprotic water miscible organic solvents upto refluxing for a period 48 hours isolating 1,1'-{[(bis alkane-1,N-diyl)piperazine]dioxy}bis[(11a*S*)-7-methoxy-2-nitrobenzoylpyrrolidin-2-carbox- aldehyde diethylthioacetal] IV where n is 2-10 by conventional methods, reducing the above nitro compounds of formula IV with SnCl₂ .2H₂O in presence of organic solvent up to a reflux temperature, isolating the 1,1'-{[(bisalkane-1,N-diyl) piperazine]dioxy}bis[(11a*S*)-7-methoxy-2-aminobenzoylpyrrolidin-2-carboxaldehyde diethyl thioacetal] of formula V where n is 2-10 by known methods, reacting the above said amino compound of formula V with known deprotecting agents in a conventional manner to give novel pyrrolo[2,1-c][1,4]benzodiazepines of formula VI wherein n are as stated above.

The precursor, (2*S*)-N- (4-hydroxy-2-methoxy-2-nitrobenzoyl) pyrrolidine-2-carboxaldehyde diethyl thioacetal of formula I (intermediates of DC-81) prepared by literature methods (Thurston, D.E.; Murthy, V. S.; Langley, D. R.; Jones, G. B. *Synthesis*, 1990,81)
Some representative compounds of formula VI present invention are given below
1) 1,1'-{[(bisethane-1,N-diyl)piperazine]dioxy}bis[(11a*S*)-7-methoxy-1,2,3, 11a-tetrahydro-5*H*-pyrrolo[2,1-*c*][1,4]benzodiazepin-5-one].
2) 1,1'-{[(bispropane-1,N-diyl)piperazine]dioxy}bis[(11a*S*)-7-methoxy-1,2,3, 11a-tetrahydro-5*H*-pyrrolo[2,1-*c*][1,4]benzodiazepin-5-one].
3) 1,1'-{[(bisbutane-1,N-diyl)piperazine]dioxy}bis[(11a*S*)-7-methoxy-1,2,3, 11a-tetrahydro-5*H*-pyrrolo[2,1-*c*][1,4]benzodiazepin-5-one].

These new analogues of pyrrolo[2,1-c][1,4]benzodiazepinedimers linked at C-8 position through piperazine moiety have shown promising anticancer activity in various cell lines. The molecules synthesized are of immense biological significance with potential sequence selective DNA-binding property. This resulted in design and synthesis of new congeners as illustrated in Scheme-1, which comprise:
1. The ether linkage at C-8 position of DC-81 intermediates with piperazine moiety
2. Refluxing the reaction mixture for 24-48 h.
3. Synthesis of C-8 linked PBD antitumour antibiotic dimer imines.
4. Purification by column chromatography using different solvents like ethylacetate, hexane, dichloromethane and methanol.
The process of preparation of new non-cross linking pyrrolo[2,1-*c*][1,4]benzodiazepines is disclosed and claimed a co-pending application of the applicant. The following examples are given by way of illustration and therefore should not be construed to the present limit of the scope of invention.

### Brief description of accompanying drawing

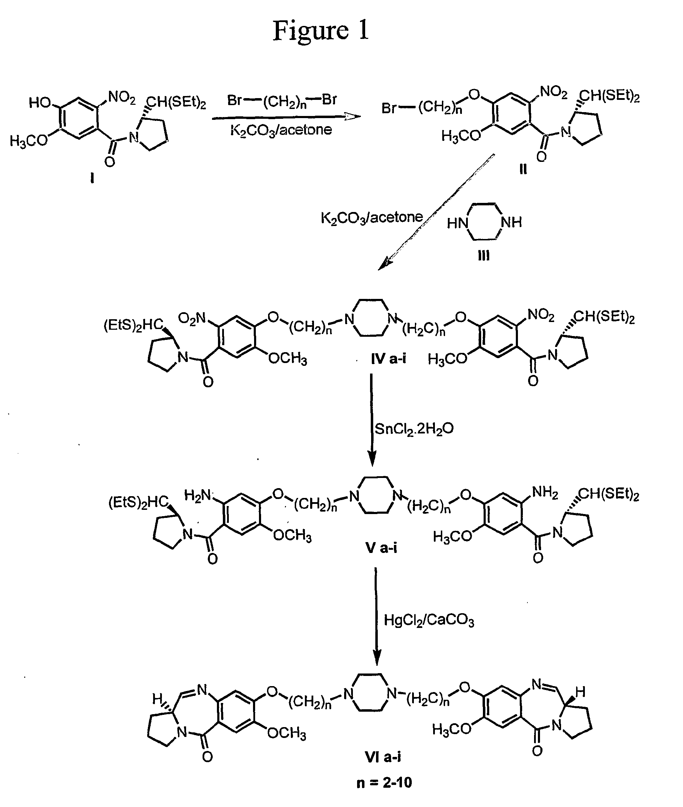

### Figure 1 represents schematic diagram of preparing compound of general formula VI (a - i).

### Example 1

A solution of (2*S*)-N-(4-hydroxy-5-methoxy-2-nitrobenzoyl)pyrrolidine-2-carboxaldehyde diethylthioacetal of formula I (800 mg, 2 mmol), 1, 2-dibromoethane (940 mg, 2.5 mmol) and K₂CO₃ (828 mg, 3 mmol) in dry acetone (40 ml) was refluxed for 48h. After the completion of reaction as indicated by TLC, EtOAc-hexane (7:3), the reaction mixture was poured on to the water and then extracted with ethylacetate. Evaporation of the organic layer gave the crude product, which was further purified by column chromatography on silica gel eluting with EtOAc-hexane (1:1) gave the pure (2*S*)-N-[4-(2-bromoethoxy)-5-methoxy-2-nitrobenzoyl]pyrrolidine-2-carboxaldehyde diethyl thioacetal of formula II.
¹H NMR: (CDCl₃) δ 1.20-1.4 (m, 6H), 1.75-2.2 (m, 4H), 2.6-2.9 (m, 4H), 3.20-3.33 (m, 2H), 3.67 (t, 2H), 3.95 (s, 3H); 4.37 (t, 2H), 4.62-4.78 (m, 1H), 4.85 (d, 1H), 6.82 (s, 1H), 7.67 (s, 1H).

A solution of (2*S*)-N-[4-(3-bromoethoxy)-5-methoxy-2-nitrobenzoyl]pyrrolidine-2-carboxaldehyde diethylthioacetal of formula II (507 mg, 1 mmol), piperazine(0.043 mg, 0.5 mmol) of the formula III and K₂CO₃ (414 mg, 3 mmol) in dry acetone (20 ml) was refluxed for 48h. After the completion of reaction as indicated by TLC, EtOAc, the reaction mixture was poured on to the water and then extracted with ethylacetate. Evaporation of the organic layer gave the crude product, which was further purified by column chromatography on silica gel eluting with EtOAc-hexane (9:1) gave the pure 1,1'-{[(bisethane-1,N-diyl)piperazine]dioxy}bis[(11aS)-7-methoxy2-nitrobenzoylpyrrolidin-2-carboxaldehyde diethylthioacetal].
¹H NMR (CDCl₃) δ 1.29-1.41 (m, 12H), 1.7-2.39 (m, 8H), 2.60-2.90 (m, 20H), 3.17-3.3 (m, 4H), 3.92 (s, 6H), 4.2 (t, 4H), 4.60-4.70 (m, 2H), 4.81(d, 2H), 6.8 (s, 2H), 7.7 (s, 2H). FAB MS 939 (M+H)⁺.

The 1,1'-{[(bisethane-1,N-diyl)piperazine]dioxy}bis[(11a*S*)-7-methoxy-2-nitrobenzoylpyrrolidin-2-carboxaldehyde diethylthioacetal] IV (939 mg, 1.0 mmol) was dissolved in methanol (10 mL) and added SnCl₂.2H₂O (1.124 g, 5.0 mmol) was refluxed for 1.5 h. The reaction mixture was then carefully adjusted to pH 8 with saturated NaHCO₃ solution and then extracted with ethyl acetate (3x20 mL). The combined organic phase was dried over Na₂SO₄ and evaporated under vacuum to afford the crude The 1,1'-{[(bisethane-1,N-diyl)piperazine]dioxy}bis[(11a*S*)-7-methoxy 2-aminobenzoylpyrrolidin-2-carboxaldehyde diethylthioacetal].

A solution of the 1,1'-{[(bisethane-1,N-diyl)piperazine]dioxy}bis[(11a*S*)-7-methoxy 2-aminobenzoylpyrrolidin-2-carboxaldehyde diethylthioacetal] of formula V (879 mg, 1 mmol), HgCl₂ (794 mg, 2.93 mmol) and HgO (686 mg, 3.18 mmol) in CH₃CN/H₂O (3:1, 15 ml) was stirred at room temperature for 12h until TLC (EtOAc), indicates complete loss of starting material. Then organic layer is evaporated in vacuum and the residue is diluted with EtOAc. To this saturated NaHCO₃ was added slowly at room temperature and the mixture is filtered through celite and washed with ethylacetate. The filterate is evaporated in vacuum to get crude 1,1'-{[(bisethane-1,N-diyl) piperazine]dioxy}bis[(11a*S*)-7-methoxy-1,2,3,11a-tetrahydro-5*H*-pyrrolo[2,1*-c*][1,4] benzodiazepin-5-one] of formula VIa, which was further purified by column chromatography on silica gel eluting first with ethylacetate to remove traces of mercuric salts and further eluted with CHCl₃-methanol (9:1).
¹HNMR (CDCl₃) δ 1.92-2.42 (m, 8H), 2.60-2.95 (m, 12H), 3.2-3.88 (m, 6H), 3.92 (s, 6H), 4.14-4.28 (m, 4H), 6.76 (s, 2H), 7.5 (s, 2H), 7.66 (d, 2H). FAB MS: 631 (M+H)⁺.

### Example 2

A solution of (2*S*)-N-(4-hydroxy-5-methoxy-2-nitrobenzoyl)pyrrolidine-2-carboxaldehyde diethylthioacetal of formula I (400 mg, 1 mmol), 1,3-dibromopropane (502 mg, 2.5 mmol) and K₂CO₃ (414 mg, 3 mmol) in dry acetone (20 ml) was refluxed for 48h. After the completion of reaction as indicated by TLC, EtOAc-hexane (7:3), the reaction mixture was poured on to the water and then extracted with ethylacetate. Evaporation of the organic layer gave the crude product, which was further purified by column chromatography on silica gel eluting with EtOAc-hexane (1:1) gave the pure (2*S*)-N-[4-(4-bromopropoxy)-5-methoxy-2-nitrobenzoyl]pyrrolidine-2-carboxaldehyde diethylthioacetal of formula II.
¹H NMR: (CDCl₃) δ 1.25-1.4 (m, 6H), 1.85-2.35 (m, 4H), 2.38-2.5 (m, 2H), 2.6-2.9 (m, 4H), 3.18-3.33 (m, 2H), 3.64 (t, 2H), 3.97 (s, 3H); 4.29 (t, 2H), 4.67-4.78 (m, 1H), 4.83 (d, 1H), 6.78 (s, 1H), 7.7 (s, 1H).

A solution of (2*S*)-N-[4-(4-bromopropoxy)-5-methoxy-2-nitrobenzoyl]pyrrolidine-2-carboxaldehyde diethylthioacetal of formula II (520 mg, 1 mmol), piperazine (0.043 mg, 1 mmol) of the formula III and K₂CO₃ (414 mg, 3 mmol) in dry acetone (20 ml) was refluxed for 48h. After the completion of reaction as indicated by TLC, EtOAc, the reaction mixture was poured on to the water and then extracted with ethylacetate. Evaporation of the organic layer gave the crude product, which was further purified by column chromatography on silica gel eluting with EtOAc-hexane (9:1) gave the pure of 1,1'-{[(bispropane-1,N-diyl)piperazine]dioxy}bis[(11a*S*)-7-methoxy-2-nitro benzoylpyrrolidin-2-carboxaldehyde diethylthioacetal].formula IV.
¹H NMR (CDCl₃) δ 1.3-1.42 (m, 12H), 1.9-2.32 (m, 8H), 2.47-2.6 (m, 4H), 2.7-2.9 (m, 24H), 3.2-3.3 (m, 4H), 3.95 (s, 6H), 4.1-4.2 (t, 4H), 4.62-4.75 (m, 2H), 4.82 (d, 2H), 6.75 (s, 2H), 7.67 (s, 2H). FAB MS: 967(M+H)⁺

The 1,1'-{[(bispropane-1,N-diyl)piperazine]dioxy}bis[(11a*S*)-7-methoxy-2-nitro benzoylpyrrolidin-2-carboxaldehyde diethylthioacetal] (966 mg, 1.0 mmol) of the formula IV was dissolved in methanol (10 ml) and added SnCl₂.2H₂O (1.124 g, 5.0 mmol) was refluxed for 1.5 h. The reaction mixture was then carefully adjusted to pH 8 with saturated NaHCO₃ solution and then extracted with ethyl acetate (3x20 ml). The combined organic phase was dried over Na₂SO₄ and evaporated under vacuum to afford the crude 1,1'-{[(bis propane-1,N-diyl)piperazine]dioxy}bis[(11a*S*)-7-methoxy 2-aminobenzoylpyrrolidin-2-carboxaldehyde diethylthioacetal] of formula V.
A solution of 1,1'-{[(bispropane-1,N-diyl)piperazine]dioxy}bis[(11a*S*)-7-methoxy 2-aminobenzoylpyrrolidin-2-carboxaldehyde diethylthioacetal]the formula V. (907 mg, 1 mmol), HgCl₂ (794 mg, 2.93 mmol) and HgO (687 mg, 3.18 mmol) in CH₃CN/H₂O (3:1, 15 ml) was stirred at room temperature for 12h until TLC (EtOAc), indicates complete loss of starting material. Then organic layer is evaporated in vacuum and the residue is diluted with EtOAc. To this saturated NaHCO₃ was added slowly at room temperature and the mixture is filtered thorough celite and washed with ethylacetate. The filterate is evaporated in vacuum to get crude 1,1'-{[(bispropane-1,N-diyl) piperazine]dioxy}bis[(11a*S*)-7-methoxy-1,2,3,11a-tetrahydro-5*H*-pyrrolo[2,1-*c*][1,4] benzodiazepin-5-one] of formula VIb, which was further purified by column chromatography on silica gel eluting first with ethylacetate to remove traces of mercuric salts and further eluted with CHCl₃-methanol (9:1).
¹HNMR¹ (CDCl₃) δ 1.92-2.37 (m, 8H), 2.57-2.8 (m, 16H), 3.32-3.75 (m, 6H), 3.95 (s, 6H) 4.12-4.45(m, 4H), 6.85 (s, 2H), 7.52 (s, 2H), 7.82 (d, 2H) FAB MS : 659 (M+H)⁺

### Example 3

A solution of (2*S*)-N-(4-hydroxy-5-methoxy-2-nitrobenzoyl)pyrrolidine-2-carboxaldehyde diethylthioacetal of formula I (400mg, 1 mmol), 1,4-dibromobutane (540 mg, 2.5 mmol) and K₂CO₃ (414 mg, 3 mmol) in dry acetone (20 ml) was refluxed for 48h. After the completion of reaction as indicated by TLC, EtOAc-hexane (7:3), the reaction mixture was poured on to the water and then extracted with ethylacetate. Evaporation of the organic layer gave the crude product, which was further purified by column chromatography on silica gel eluting with EtOAc-hexane (1:1) gave the pure (2*S*)-N-[4-(5-bromobutanoxy)-5-methoxy-2-nitrobenzoyl]pyrrolidine-2-carboxaldehyde diethylthioacetal of formula II.
¹H NMR: (CDCl₃) δ 1.3-1.45(m, 6H), 1.88-2.38 (m, 4H), 2.69-2.88 (m, 8H), 3.20-3.33 (m, 2H), 3.51 (t, 2H), 3.97 (s, 3H); 4.16 (t, 2H), 4.63-4.76 (m, 1H), 4.86(d, 1H), 6.79 (s, 1H), 7.67 (s, 1H).

A solution of (2*S*)-N-[4-(5-bromobutanoxy)-5-methoxy-2-nitrobenzoyl]pyrrolidine-2-carboxaldehyde diethylthioacetal of formula II. (53 mg, 1 mmol), piperazine(0.043 mg, 1 mmol) of formula III and K₂CO₃ (414 mg, 3 mmol) in dry acetone (20 ml) was refluxed for 48h. After the completion of reaction as indicated by TLC, EtOAc, the reaction mixture was poured on to the water and then extracted with ethylacetate. Evaporation of the organic layer gave the crude product, which was further purified by column chromatography on silica gel eluting with EtOAc-hexane (9:1) gave the pure of 1,1'-{[(bisbutane-1,N-diyl)piperazine]dioxy}bis[(11a*S*)-7-methoxy-2-nitro benzoylpyrrolidin-2-carboxaldehyde diethylthioacetal] of formula IV.
¹H NMR (CDCl₃) δ 1.30-1.43 (m, 12H), 2.74-2.35 (m, 12H), 2.51-2.66 (m, 16H), 3.20-3.3 (m, 4H), 3.97 (s, 6H), 4.12 (t, 4H), 4.64-4.76 (m, 2H), 4.87 (d, 2H), 6.84 (s, 2H), 7.66 (s, 2H).
FAB MS: 995 (M+H)⁺

The of 1,1'-{[(bisbutane-1,N-diyl)piperazine]dioxy}bis[(11a*S*)-7-methoxy-2-nitro benzoylpyrrolidin-2-carboxaldehyde diethylthioacetal] of formula IV (730 mg, 1.0 mmol) was dissolved in methanol (10 ml) and added SnCl₂.2H₂O (1.124 g, 5.0 mmol) was refluxed for 1.5 h. The reaction mixture was then carefully adjusted to pH 8 with saturated NaHCO₃ solution and then extracted with ethyl acetate (3x20 ml). The combined organic phase was dried over Na₂SO₄ and evaporated under vacuum to afford the crude of 1,1'-{[(bisbutane-1,N-diyl) piperazine]dioxy}bis[(11a*S*)-7-methoxy-2-aminobenzoylpyrrolidin-2-carboxaldehyde diethylthioacetal].of formula V.

A solution of 1,1'-{[(bisbutane-1,N-diyl)piperazine]dioxy}bis[(11a*S*)-7-methoxy 2-aminobenzoylpyrrolidin-2-carboxaldehyde diethylthioacetal] formula V. (935 mg, 1 mmol), HgCl₂ (794 mg, 2.93 mmol) and HgO (687 mg, 3.18 mmol) in CH₃CN/H₂O (3:1, 15 ml) was stirred at room temperature for 12h until TLC (EtOAc), indicates complete loss of starting material. Then organic layer is evaporated in vacuum and the residue is diluted with EtOAc. To this saturated NaHCO₃ was added slowly at room temperature and the mixture is filtered thorough celite and washed with ethylacetate. The filterate is evaporated in vacuum to get crude 1,1'-{[(bisbutane-1,N-diyl) piperazine]dioxy}bis[(11a*S*)-7-methoxy-1,2,3,11a-tetrahydro-5*H*-pyrrolo[2,1-*c*][1,4] benzodiazepin-5-one] VIc, which was further purified by column chromatography on silica gel eluting first with ethylacetate to remove traces of mercuric salts and further eluted with CHCl₃-methanol (9:1).
¹HNMR (CDCl₃) δ 1.78-2.24 (m, 8H), 2.30-2.75 (m, 20H), 3.4-3.7 (m, 6H), 3.92 (s, 6H), 4.1-4.23 (m, 4H), 6.73 (s, 2H), 7.48 (s, 2H), 7.60 (d, 2H).
FAB MS 687 (M+H)⁺
**Biological Activity**: *In vitro* biological activity studies were carried out at National Cancer Institute (USA).
Cytotoxicity: Compounds VIa-d were evaluated in vitro against sixty human tumour cells derived from nine cancer types (leukemia, non-small-cell lung, colon, CNS, melanoma, ovarian, prostate, and breast cancer). For each compound, dose response curves for each cell line were measured at a minimum of five concentrations at 10 fold dilutions. A protocol of 48 h continuous drug exposure was used, and a sulforhodamine B (SRB) protein assay was used to estimate cell viability or growth. The concentration causing 50 % cell growth inhibition (GI50), total cell growth inhibition (TGI, 0% growth) and 50% cell death (LC50, -50% growth) compared with the control was calculated. The mean graph midpoint values of log₁₀TGI and log₁₀LC50 as well as log₁₀ GI50 for VIa-d are listed in Table 1. As demonstrated by mean graph pattern, compound VIc exhibits an interesting profile of activity and selectivity for various cell lines. The mean graph mid point of log₁₀ TGI and log₁₀ LC50 showed similar pattern to the log₁₀ GI50 mean graph mid points.

**Table 1. Log₁₀ GI50 log₁₀ TGI and log₁₀ LC50 mean graphs midpoints (MG_MID) of in vitro Cytotoxicity data for the compounds VI a-d against human tumor cell lines.**

| **Compound** | **Log₁₀ GI50** | **Log₁₀ TGI** | **Log₁₀ LC50** |
|---|---|---|---|
| VIa | -4.69 | -4.16 | -4.03 |
| VIb | -5.19 | -4.22 | -4.01 |
| VIc | -7.70 | -5.95 | -4.47 |
| VId | -5.14 | -4.26 | -4.04 |

The *in vitro* anticancer activity for four representative compounds has been given in **Table 2**. The comparison of the data of Table 2 reveals the importance of the alkane spacer. As the alkane spacer increased from 2-4 the cytotoxic activity has moderately enhanced. The 4-carbon spacer of compound VIc confers a suitable fit in the minor groove of double helix DNA and show slightly higher activity in this series of compounds VI a-d.

**Table 2. Log LC50 (concentration in mol/L causing 50% lethality) Values for Compounds VI a-d**

| **Cancer** | **Compound VIa** | | **Compound VIb** | | **Compound VIc** | | **Compound VId** | |
|---|---|---|---|---|---|---|---|---|
| | **GI 50** | **LC 50** | **GI 50** | **LC 50** | **GI 50** | **LC 50** | **GI 50** | **LC 50** |
| **Leukemia** | | | | | | | | |
| HL-60(TB) | -5.49 | -4.00 | -6.66 | -4.00 | -8.00 | -4.00 | -5.38 | -4.00 |
| K-62 | -4.58 | -4.00 | -5.76 | -4.00 | -7.63 | -4.00 | -5.46 | -4.20 |
| MOLT-4 | -5.68 | -4.10 | -6.50 | -4.08 | -8.00 | -4.00 | -4.79 | -4.00 |
| RPMI-8226 | -6.08 | -4.33 | -6.73 | -4.00 | -7.81 | -4.60 | -5.59 | -4.54 |
| SR | -6.68 | -5.21 | - | - | -8.00 | -5.62 | -5.37 | -4.00 |

| **Non-small-cell lung** | | | | | | | | |
|---|---|---|---|---|---|---|---|---|
| A549/ATCC | -4.10 | -4.00 | -4.37 | -4.00 | -7.23 | -4.00 | -4.66 | -4.00 |
| EKVX | -4.00 | -4.00 | -4.47 | -4.00 | -6.36 | -4.00 | -4.33 | -4.00 |
| HOP-62 | -4.46 | -4.00 | -5.14 | -4.00 | -8.00 | -4.00 | -4.00 | -4.00 |
| HOP-92 | -4.68 | -4.00 | -6.81 | -4.00 | -8.00 | -4.00 | -5.46 | -4.00 |
| NCI-H226 | -4.80 | -4.00 | -4.34 | -4.00 | -8.00 | -4.00 | -5.30 | -4.00 |
| NCI-H23 | -4.76 | -4.00 | -4.89 | -4.00 | -8.00 | -5.59 | -5.18 | -4.00 |
| NCI-H322 | -4.51 | -4.00 | -4.57 | -4.00 | -7.59 | -4.00 | -4.65 | -4.00 |
| NCI-H460 | -4.95 | -4.00 | -5.37 | -4.00 | -8.00 | -4.00 | -5.49 | -4.00 |
| NCI-H522 | -4.94 | -4.00 | -6.40 | -4.00 | -8.00 | -4.00 | -5.47 | -4.00 |

| **Colon** | | | | | | | | |
|---|---|---|---|---|---|---|---|---|
| COLO 205 | -4.66 | -4.00 | -5.34 | -4.00 | -7.99 | -6.26 | -5.49 | -4.00 |
| HCC-2998 | - | - | -4.73 | -4.04 | -7.95 | -4.00 | -5.69 | -4.29 |
| HCT-116 | -4.47 | -4.00 | -4.92 | -4.00 | -6.42 | -4.00 | -4.00 | -4.00 |
| HCT-15 | -4.39 | -4.00 | -4.25 | -4.00 | -7.92 | -6.37 | -4.25 | -4.00 |
| HT-29 | -4.58 | -4.00 | -4.46 | -4.00 | -7.89 | -4.00 | -5.02 | -4.00 |
| KM-12 | -4.70 | -4.04 | -4.64 | -4.00 | -7.84 | -4.67 | -5.25 | -4.00 |
| SW-620 | -4.68 | -4.00 | -6.23 | -4.00 | - | - | -5.49 | -4.00 |

| **CNS** | | | | | | | | |
|---|---|---|---|---|---|---|---|---|
| SF-268 | -4.95 | -4.00 | -5.52 | 4.00 | -8.00 | -4.00 | -5.44 | -4.00 |
| SF-295 | -5.06 | -4.00 | -4.99 | -4.00 | -8.00 | -4.69 | -5.30 | -4.00 |
| SF-539 | -4.97 | -4.00 | -6.21 | -4.00 | -8.00 | -4.00 | -5.44 | -4.00 |
| SNB-19 | -4.75 | -4.00 | -5.06 | -4.00 | -8.00 | -4.44 | -4.00 | -4.00 |
| SNB-75 | -4.23 | -4.00 | -4.61 | -4.00 | -7.94 | -4.00 | -4.51 | -4.00 |
| U251 | -4.87 | -4.00 | -5.41 | -4.00 | -8.00 | -4.41 | -5.47 | -4.00 |

| **Melanoma** | | | | | | | | |
|---|---|---|---|---|---|---|---|---|
| MALME- | -5.46 | -4.17 | -5.39 | 4.00 | -8.00 | -7.41 | - | - |
| 3M | - | - | - | - | - | - | -5.61 | -4.00 |
| LOXIMVI | -4.61 | 4.00 | -5.55 | -4.00 | -7.60 | -4.17 | -4.76 | -4.00 |
| M14 | -4.57 | -4.00 | -4.82 | -4.00 | -7.34 | -4.00 | - | - |
| SK-MEL-2 | -4.22 | -4.00 | -4.48 | -4.00 | -7.69 | -4.00 | -4.71 | -4.00 |
| SK-MEL-28 | -4.75 | -4.00 | -5.66 | -4.50 | -7.86 | -6.68 | -5.48 | -4.00 |
| SK-MEL-5 | -4.49 | -4.00 | -4.45 | -4.00 | -7.65 | - | -4.75 | -4.00 |
| UACC-257 | -4.83 | -4.00 | -4.68 | -4.00 | -8.00 | -7.35 | -5.64 | -4.00 |
| UACC-62 | | | | | | | | |

| **Ovarian** | | | | | | | | |
|---|---|---|---|---|---|---|---|---|
| IGROVI | -4.21 | -4.00 | -5.26 | -4.00 | -6.79 | -4.00 | -5.13 | -4.00 |
| OSCAR-3 | -4.68 | -4.00 | -5.47 | -4.00 | -7.91 | -4.00 | -5.32 | -4.00 |
| OVCAR-4 | -4.00 | -4.00 | -4.13 | -4.00 | -7.11 | -4.00 | -5.38 | -4.00 |
| OVCAR-5 | -4.65 | -4.00 | -5.06 | -4.00 | -7.92 | -4.00 | -5.33 | -4.00 |
| OVCAR-8 | -4.58 | -4.00 | -4.48 | -4.00 | -8.00 | -4.00 | -4,67 | -4.00 |
| SK-OV-3 | -4.00 | -4.00 | - | - | - | -4.00 | - | - |

| **Renal** | | | | | | | | |
|---|---|---|---|---|---|---|---|---|
| 786-0 | -4.84 | -4.00 | -5.30 | -4.00 | -8.00 | -4.00 | -5.61 | -4.00 |
| A498 | -4.29 | -4.00 | -5.73 | -4.00 | -6.89 | - | -5.00 | -4.00 |
| ACHN | -4.82 | -4.00 | -4.47 | -4.00 | -8.00 | -4.00 | -4.63 | -4.00 |
| CAKI-1 | -5.04 | -4.00 | -4.65 | -4.00 | -8.00 | -4.28 | -5.77 | -4.00 |
| RXF 393 | -4.23 | -4.00 | -5.68 | -4.09 | -7.54 | -4.00 | -8.00 | -5.09 |
| SN12C | -4.90 | -4.00 | -4.44 | -4.00 | -8.00 | -4.00 | -5.66 | -4.00 |
| TK-10 | -4.63 | -4.00 | -5.10 | -4.00 | -7.69 | -4.00 | - | -4.00 |
| UO-31 | -4.12 | -4.00 | -4.27 | -4.00 | -6.65 | -4.00 | -4.00 | -4.00 |

| **Prostate** | | | | | | | | |
|---|---|---|---|---|---|---|---|---|
| PC-3 | -4.44 | -4.00 | -5.53 | -4.00 | -7.02 | -4.00 | -5.41 | -4.00 |
| DU-145 | -4.36 | -4.00 | -5.62 | -4.00 | -7.60 | -4.00 | -5.37 | -4.00 |

| **Breast** | | | | | | | | |
|---|---|---|---|---|---|---|---|---|
| MCF7 | -4.88 | -4.00 | -6.01 | -4.00 | -8.00 | -4.00 | -5.69 | -4.00 |
| NCI/ADR-RES | -4.00 | -4.00 | -4.00 | -4.00 | -6.47 | -4.00 | -4.00 | -4.00 |
| MDA-MB-231/ATCC | -4.67 | -4.00 | -4.52 | -4.00 | -7.47 | -4.43 | -5.42 | -4.00 |
| HS578T | -4.34 | -4.00 | -6.13 | -4.00 | -7.30 | -4.00 | -5.12 | -4.00 |
| MDA-MB-435 | -4.71 | -4.08 | -5.37 | -4.09 | -7.87 | -7.15 | -5.37 | -4.00 |
| BT-549 | -4.56 | -4.00 | -5.77 | -4.00 | -7.68 | -4.00 | -5.31 | -4.00 |
| T-47D | -4.57 | -4.00 | -5.16 | -4.00 | -8.00 | -4.00 | -5.00 | -4.00 |

## Claims

1. Analogues of 1,1'-{[(bisalkane-1,N-diyl)piperazine]dioxy}bis(11aS)-7-methoxy-1,2,3,11a-tetrahydro-5H-pyrrolo[2,1-c] [1,4]benzodiazepin-5-one] of formula (VI) 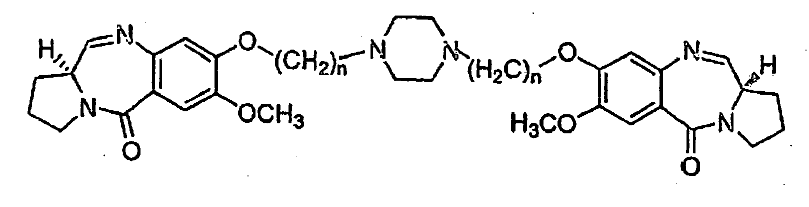 where n = 2 to 10.

2. A process for the preparation of compounds of claim 1, the said process comprising steps of:
a) reacting compound of formula (I) 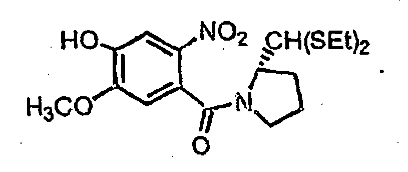 with 1, 2-dibromoethane in water miscible organic solvent in presence of a base at a reflux temperature for a period of 20h to 48h,
b) pouring the reaction mixture of step (a) onto water, extracting with ethylacetate separating ethylacetate layer and discarding aqueous layer,
c) evaporating the ethylacetate layer of step (b) to obtain a residue which is further purified to obtain pure compound of formula (II), 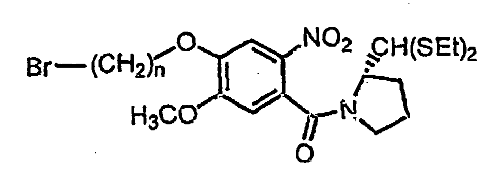
d) providing a solution of formula (II) in aketonic solvent in presence of a base at a reflux temperature for a period of 20 h to 48 h,
e) pouring the reaction mixture of step (d) onto water, extracting with ethylacetate, separating ethylacetate layer, evaporating ethylacetate layer to obtain a residue, purifying the residue to get compound of formula (IV), 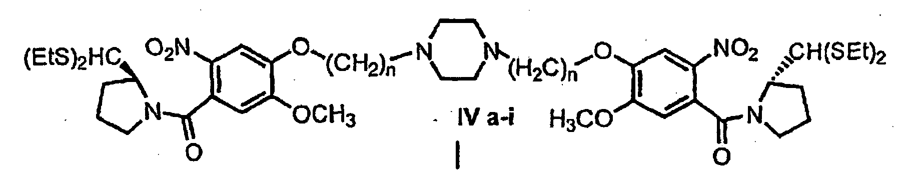
f) dissolving compound of formula (IV) in alcohol, adding stannous chloride dihydrate, refluxing for 0.5 h tol.5h,
g) adjusting the pH of the reaction mixture of step(f) to 8.0 using alkali bicarbonate solution,
h)extracting solution of pH 8.0 of step (g) with ethylacetate, separating ethylacetate extract, drying ethylacetate extract over anhydrous sodium sulphate, filtered and evaporated ethyl acetate solution to yield a crude compound of formula (V), 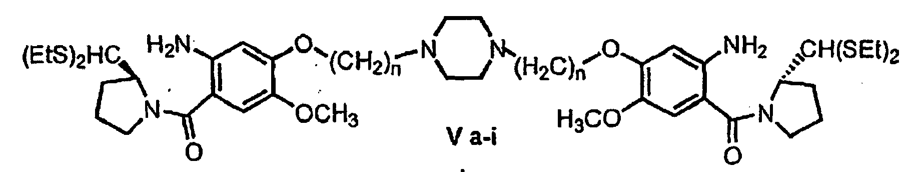
i) dissolving compound of formula (V) of step (h) in a mixture of acetonitrile/water, adding mercuric chloride, mercuric oxide and stirred for 6h to 12 h at an ambient temperature,
j) evaporating organic layer of step (i), diluting the residue with ethylacetate, adding saturated bicarbonate solution at room temperature, filtering through celite bed, washed with ethyl acetate to obtain a clear filterate; and
k) evaporating filtrate of step (j) to obtain a residue which is purified over silica gel column to yield pure compound of formula (VI), according to claim 1.

3. A process of claim 2, wherein in step (a) the base used is selected from a group consisting of lithium carbonate, sodium carbonate, potassium carbonate or cesium carbonate.

4. A process of claim 2, wherein in step (d) the ketonic solvent used is selected from a group consisting of acetone, methyl ethyl ketone and methyl isobutylketone.

5. A process of claim 2, wherein in step (d) the base used is selected from a group consisting of lithium carbonate, sodium carbonate, potassium carbonate and cesium carbonate.

6. A process of claim 2, wherein in step (f) the alcohol used, is selected from methanol, ethanol and isopropanol.

7. A process of claim 6, wherein the alcohol used is methanol.

8. A pharmaceutical composition useful as anti-tumor agent, said composition comprising an effective amount of one or more analogues of 1,1'-{[(bisalkane-1, N-diyl) piperazine] dioxy} bis (11aS)-7-methoxy-1,2,3,11a-tetrahydro-SH-pyrrolo [2,1-c][1,4]benzodiazepin-5-one] of formula (VI), according to claim1.

9. A composition as claimed in claim 8, wherein the composition optionally comprising pharmaceutically acceptable additives.

10. A composition as claimed in claim 8, wherein the composition is administered to mammals including human beings.

11. A composition as claimed in claim 10, wherein the composition is administered orally, systemically or by any other conventional methods.

## Patentansprüche

1. Analoga von 1,1'-{[(Bisalkan-1,N-diyl)piperazin]dioxy}-bis[(11aS)-7-methoxy-1,2,3,11a-tetrahydro-5H-pyrrolo[2,1-c][1,4]benzodiazepin-5-on] der Formel (VI) 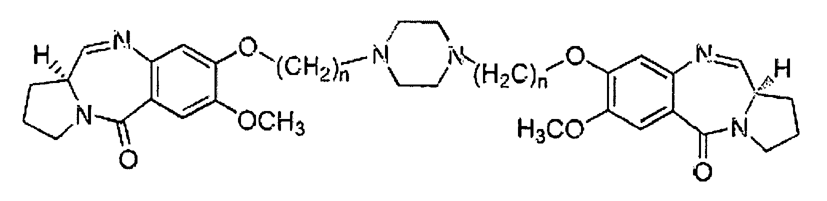 worin n = 2 bis 10.

2. Verfahren zur Herstellung von Verbindungen nach Anspruch 1, wobei das Verfahren die folgenden Stufen umfasst:
a) Umsetzen einer Verbindung der Formel (I) 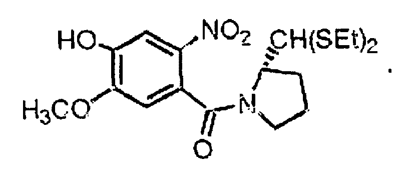 mit 1,2-Dibromethan in einem wassermischbaren organischen Lösungsmittel in Gegenwart einer Base bei einer Rückflusstemperatur für eine Dauer von 20 Stunden bis 48 Stunden,
b) Gießen des Reaktionsgemisches aus Stufe (a) auf Wasser, Extrahieren mit Ethylacetat, Abtrennen der Ethylacetatschicht und Verwerfen der wässrigen Schicht,
c) Eindampfen der Ethylacetatschicht aus Stufe (b) unter Erhalt eines Rückstands, der weiter gereinigt wird, um die reine Verbindung der Formel (II) zu erhalten, 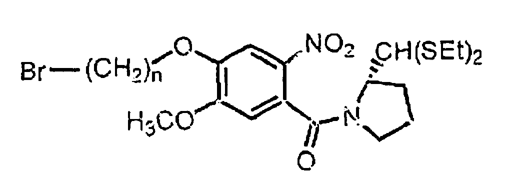
d) Bereitstellen einer Lösung der Formel (II) in einem Ketonlösungsmittel in Gegenwart einer Base bei einer Rückflusstemperatur für eine Dauer von 20 Stunden bis 48 Stunden,
e) Gießen des Reaktionsgemisches aus Stufe (d) auf Wasser, Extrahieren mit Ethylacetat, Abtrennen der Ethylacetatschicht, Eindampfen der Ethylacetatschicht unter Erhalt eines Rückstands, Reinigen des Rückstands unter Erhalt einer Verbindung der Formel (IV), 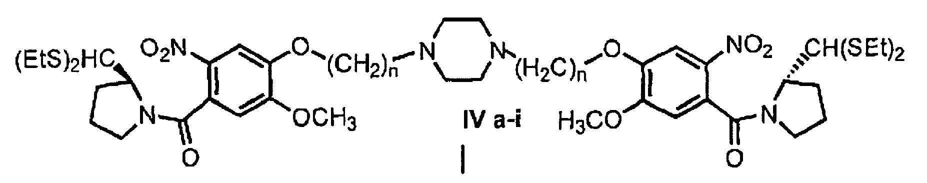
f) Auflösen der Verbindung der Formel (IV) in Alkohol, Zugeben von Zinn(II)-chlorid-Dihydrat, Erhitzen unter Rückfluss für 0,5 Stunden bis 1,5 Stunden,
g) Einstellen des pH des Reaktionsgemisches von Stufe (f) auf 8,0 unter Verwendung einer Alkalihydrogencarbonatlösung,
h) Extrahieren der Lösung mit pH 8,0 aus Stufe (g) mit Ethylacetat, Abtrennen des Ethylacetatextraktes, Trocknen des Ethylacetatextraktes über wasserfreiem Natriumsulfat, wobei eine filtrierte und eingedampfte Ethylacetatlösung eine rohe Verbindung der Formel (V) ergibt, 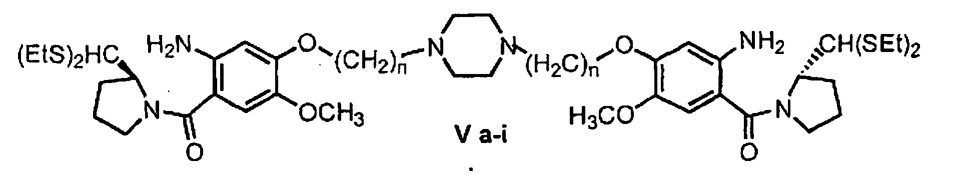
i) Auflösen der Verbindung der Formel (V) aus Stufe (h) in einem Gemisch aus Acetonitril/Wasser, Zugeben von Quecksilber(II)-chlorid, Quecksilber(II)-oxid und gerührt für 6 Stunden bis 12 Stunden bei einer Umgebungstemperatur,
j) Eindampfen der organischen Schicht aus Stufe (i), Verdünnen des Rückstands mit Ethylacetat, Zugeben von gesättigter Hydrogencarbonatlösung bei Raumtemperatur, Filtrieren durch ein Celite-Bett, gewaschen mit Ethylacetat, unter Erhalt eines klaren Filtrates; und
k) Eindampfen des Filtrates aus Stufe (j) unter Erhalt eines Rückstands, der über eine Silicagelsäule unter Erhalt der reinen Verbindung der Formel (VI), gemäß Anspruch 1, gereinigt wird.

3. Verfahren nach Anspruch 2, wobei die in Stufe (a) verwendete Base ausgewählt ist aus einer Gruppe, bestehend aus Lithiumcarbonat, Natriumcarbonat, Kaliumcarbonat und Cäsiumcarbonat.

4. Verfahren nach Anspruch 2, wobei das in Stufe (d) verwendete Ketonlösungsmittel ausgewählt ist aus einer Gruppe, bestehend aus Aceton, Methylethylketon und Methylisobutylketon.

5. Verfahren nach Anspruch 2, wobei die in Stufe (d) verwendete Base ausgewählt ist aus einer Gruppe, bestehend aus Lithiumcarbonat, Natriumcarbonat, Kaliumcarbonat und Cäsiumcarbonat.

6. Verfahren nach Anspruch 2, wobei der in Stufe (f) verwendete Alkohol ausgewählt ist aus Methanol, Ethanol und Isopropanol.

7. Verfahren nach Anspruch 6, wobei der verwendete Alkohol Methanol ist.

8. Pharmazeutische Zusammensetzung, die als Antitumormittel zweckmäßig ist, wobei die Zusammensetzung eine wirksame Menge von einem oder mehreren Analoga von 1,1'-{[(Bisalkan-1,N-diyl)piperazin]dioxy}-bis[(11aS)-7-methoxy-1,2,3,11a-tetrahydro-5H-pyrrolo[2,1-c][1,4]benzodiazepin-5-on] der Formel (VI), gemäß Anspruch 1, umfasst.

9. Zusammensetzung, wie in Anspruch 8 beansprucht, wobei die Zusammensetzung gegebenenfalls pharmazeutisch verträgliche Additive umfasst.

10. Zusammensetzung, wie in Anspruch 8 beansprucht, wobei die Zusammensetzung an Säuger, einschließlich Menschen, verabreicht wird.

11. Zusammensetzung, wie in Anspruch 10 beansprucht, wobei die Zusammensetzung oral, systemisch oder mittels beliebiger anderer konventioneller Verfahren verabreicht wird.

## Revendications

1. Analogues de 1,1'-{[bisalkane-1,N-diyl)piperazine]dioxy}bis(11aS)-7-methoxy-1,2,3,11a-tetrahydro-5H-pyrrolo[2,1-c][1,4]benzodiazepin-5-one] de formule (VI) 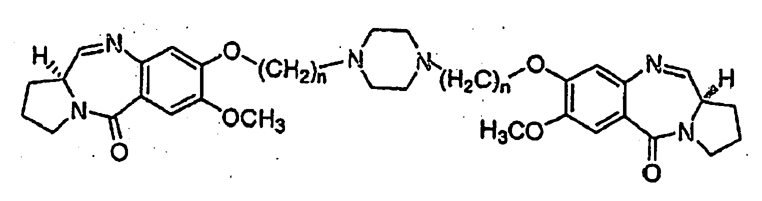 dans laquelle n = 2 à 10.

2. Un procédé pour la fabrication des composés selon la revendication 1, comprenant les étapes consistant
a) à faire réagir le composé de formule (I) 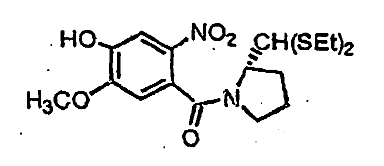 avec le dibromoéthane-1, 2 dans un solvant organique miscible avec l'eau en présence d'une base à la température de reflux pendant 20h à 48h;
b) à verser le mélange réactionnel de l'étape (a) dans de l'eau, à extraire avec de l'acétate d'éthyle, à séparer la couche de l'acétate d'éthyle et à rejeter la couche aqueuse;
c) à évaporer la couche de l'acétate d'éthyle de l'étape (b) pour obtenir un résidu qui est purifié pour obtenir le composé pur de formule (II); 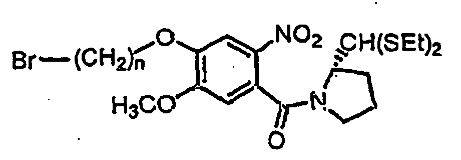
d) à préparer une solution de formule (II) dans un solvant cétonique en présence d'une base à la température de reflux pendant 20h à 48h;
e) à verser le mélange réactionnel de l'étape (d) dans de l'eau, à extraire avec de l'acétate d'éthyle, à séparer la couche de l'acétate d'éthyle, à évaporer la couche de l'acétate d'éthyle pour obtenir un résidu, à purifier le résidu pour obtenir le composé de formule (IV); 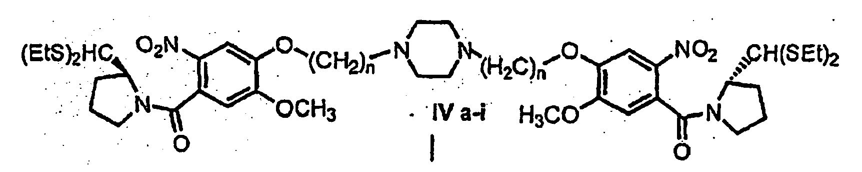
f) à dissoudre le composé de formule (IV) dans de l'alcool, à ajouter du chlorure stanneux dihydraté, à refluxer pendant 0, 5h à 1, 5h ;
g) à ajuster le pH du mélange réactionnel de l'étape (f) à 8,0 en utilisant une solution d'un hydrogénocarbonate de métal alcalin;
h) à extraire la solution avec un pH de 8,0 de l'étape (g) avec de l'acétate d'éthyle, à séparer l'extrait de l'acétate d'éthyle, à sécher l'extrait de l'acétate d'éthyle sur le sulfate de sodium anhydre, à filtrer et évaporer la solution de l'acétate d'éthyle pour obtenir le composé brut de formule (V); 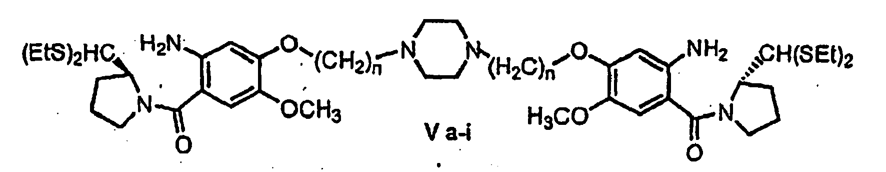
i) à dissoudre dans un mélange acétonitrile/eau le composé de formule (V) de l'étape (h), à ajouter du chlorure mercurique, de l'oxyde mercurique et à ajouter pendant 6h à 12h à la température ambiante;
j) à évaporer la couche organique de l'étape (i), à diluer le résidu avec de l'acétate d'éthyle, à ajouter d'une solution saturée de hydrogénocarbonate à la température ambiante, à filtrer à travers un lit de célite, à laver avec de l'acétate d'éthyle pour obtenir un filtrat clair; et
k) à évaporer le filtrat de l'étape (j) pour obtenir un résidu qui est purifié sur une colonne à gel de silice pour obtenir le composé pur de formule (VI) selon la revendication 1.

3. Le procédé selon la revendication 2, dans lequel la base utilisée dans l'étape (a) est choisie dans le groupe consistant en le carbonate de lithium, le carbonate de sodium, le carbonate de potassium et le carbonate de césium.

4. Le procédé selon la revendication 2, dans lequel le solvant cétonique utilisé dans l'étape (d) est choisi dans le groupe consistant en l'acétone, la méthyléthylcétone et la méthylisobutylcétone.

5. Le procédé selon la revendication 2, dans lequel la base utilisée dans l'étape (d) est choisie dans le groupe consistant en le carbonate de lithium, le carbonate de sodium, le carbonate de potassium et le carbonate de césium.

6. Le procédé selon la revendication 2, dans lequel l'alcool utilisé dans l'étape (f) est choisi parmi le méthanol, l'éthanol et l'isopropanol.

7. Le procédé selon la revendication 6, dans lequel l'alcool utilisé est le méthanol.

8. Composition pharmaceutique utilisable en tant qu'anticancéreux ladite composition comprenant une quantité efficace d'un analogue ou plusieurs analogues de 1,1'-{[bisalkane-1,N-diyl)piperazine]dioxy}bis(11aS)-7-methoxy-1,2,3,11a-tetrahydro-5H-pyrrolo[2,1-c][1,4]benzodiazepin-5-one] de formule (VI) selon la revendication 1.

9. Composition selon la revendication 8, comprenant le cas échéant des additifs pharmaceutiquement acceptables.

10. Composition selon la revendication 8, qui est administrée aux mammifères, y compris les êtres humains.

11. Composition selon la revendication 10, qui est administrée par voie orale, par voie systémique ou par une autre procédure conventionnelle.
